# EUROPEAN PATENT APPLICATION

(11) **EP 1 860 179 A1**
(43) Date of publication of application: **28.11.2007**
(21) Application number: 06729392.8
(22) Date of filing: 17.03.2006
(51) Int. Cl.: C12M 1/34, C12Q 1/02

(54) **CELL OBSERVATION AIDING INSTRUMENT AND METHOD OF CELL OBSERVATION THEREWITH**

(30) Priority: 18.03.2005 JP 2005080095
(71) Applicant: Effector Cell Institute, Inc., Tokyo 153-0041 (JP)
(72) Inventor: NITTA, Nao, home, Meguro-ku, Tokyo, 1530041 (JP); KANEGASAKI, Shiro, home, Meguro-ku, Tokyo, 1530041 (JP); YAMAUCHI, Akira, home, Meguro-ku, Tokyo, 1530041 (JP)
(74) Representative: Hackney, Nigel John
(86) International application number: PCT/JP2006/305403
(87) International publication number: WO 2006/101051

(57) **Abstract**

A cell observation support instrument having opposed first and second surface members 115 and 117 and stoppers that are positioned between the first and second surface members 115 and 117 in order to prevent movement of cells. The first and second surface members 115 and 117 form a cell-containing space 110 between opposed surfaces of the members 115 and 117, in order to contain a plurality of cells while allowing movement of the cells. At least one of the first and second surface members 115 and 117 is a member through which an inner side of the cell-containing space 110 can be optically observed from the outside. The stoppers are arranged in the cell-containing space 110, and the number of the stoppers is one or more.

## Description

### Technical Field

The present invention relates to a cell observation support instrument used for observing cells and a cell observation method using the same.

### Background Art

A cell observation support instrument (Non-Patent Document 1) for observing cells optically exists. This instrument is made by opposing a glass plate to a silicon chip provided with an etched engraving so that a cell-containing area is formed between the opposed surfaces of the glass plate and the chip. Cells are dispersed flatly in the cell-containing area such that the cells can be observed by an optical observation device such as a microscope.

By using the cell observation support instrument, it is possible to observe moving ability of cells, reaction to a stimulating substance, effects of a concentration gradient of a stimulating substance, and the like.

In making an observation of cells, it is desired that the cells are dispersed and placed within the range of the observation and observed in a fixed state at their respective positions. The technique described in Patent Document 1 provides a device for that purpose. According to this technique, surface members form a cell-containing space, and one of the surface members is provided with through-holes. Each through-hole is connected to a suction unit and cells are sucked through the through-holes such that the cells are placed and fixed on the observation surface.

Non-Patent Document 1: Kanegasaki S et al. , (2003), J. Immunol. Methods 282, 1-11
Patent Document 1: Patent No.2747304

### Disclosure of the Invention

In observing cells, sometimes it is desired that observation be continued in a state in which the cells are not injured, without changing position. In the case of the technique described in Non-Patent Document 1, it is difficult to fix cells when the cells move together with movement of liquid that fills the space containing the cells or when the cells moves in a specific direction in response to existence of a stimulating substance, for example. On the other hand, in the case of the technique described in Patent Document 1, cells can be dispersed on the observing surface by sucking the cells through the through-holes. Further, when the depth of the cell containing space is sufficiently smaller than the diameters of the cells, it is possible also to fix the cells by friction within or at the end portion of the cell-containing space. However, when it is tried to disperse cells two-dimensionally, the cells are placed at random. It is difficult to control dispersion of cells. Thus, for example, when an experiment is repeated, arrangement of cells is different each time. Further, in the case where a stimulating substance is given to cells in the cell receiving space, diffusion and fluidity of the stimulating substance is affected by the distribution of the cells. Thus, when the arrangement of the cells is random, it is very difficult to estimate the concentration of the stimulating substance affecting each cell. Further, another problem is that movement of cells by suction in the space having frictional resistance causes large loads on the cells.

An object of the present invention is to provide a technique for supporting observation of cells in a state in which the cells are arranged in desired positions with good reproducibility without affecting flow of liquid filling a space containing cells and dispersion of a substance in that space, and without applying a large load to the cells.

The present invention provides a cell observation support instrument comprising:
a first surface member and a second surface member, both members being opposed to each other; and
stoppers that are positioned between the first surface member and the second surface member, and prevent movement of cells; wherein
the first surface member and the second surface member form a cell-containing space between opposed surfaces of the first and second surface members, to contain a plurality of cells while allowing movement of the cells;
at least one of the first and second surface members is a member through which an inner side of the cell-containing space inside can be optically observed from outside; and
one or more of the stoppers is arranged inside the cell-containing space.

Further, the present invention provides a cell observation method for optically observing cells by filling a liquid into a cell-containing space formed by opposed first and second surface members, introducing the cells into the liquid, and observing the cells through a transparent member from among the first surface member and the second surface member, wherein:
one or more stoppers are arranged between the first and second surface members, in order to prevent movement of the cells; and
a plurality of cells is introduced into the cell-containing space to observe cells whose movements have been prevented by any of the stoppers.

The stoppers each may have a column structure of a size that prevents passage of at least one cell, and the column structure may have a liquid passing part that allows passage of liquid without allowing passage of a cell.

The column structure may have a curved portion that is concave toward the upstream side of flow of the cells.

The height of the stoppers may be equal to the distance between the first and second surface members. Alternatively, the height may be shorter than the distance between the first and second surface members. Alternatively, the height of the stoppers may be equivalent to cell diameter of the cells to be observed.

The stoppers may be arranged in at least one row.

The stoppers may be arranged in a plurality of rows to form a staggered array.

### Brief Description of Drawings

Fig. 1(a) is an explanatory view showing a schematic construction of a cell observation support instrument according to one embodiment of the present invention, and Fig. 1(b) is an explanatory view showing a comparative example for making the function of the embodiment clear;
Figs. 2(a) and 2(b) are perspective views seen from a bottom side, showing an example of a stopper for trapping cells in the embodiment of the present invention;
Figs. 3(a) through 3(c) are explanatory views showing a principle of preventing movement of cells by a stopper in the embodiment of the present invention;
Figs. 4(a) through 4(e) are plan views showing various forms of a stopper used in the embodiment of the present invention;
Fig. 5(a) is an explanatory view showing another form of a stopper used in the embodiment of the present invention, and Fig. 5(b) an explanatory view showing a state in which two cells are trapped by the stopper;
Figs. 6(a) through 6(c) are explanatory views showing arrangements of stoppers;
Figs. 7(a) and 7(b) are explanatory views showing examples of a combination-type stopper;
Figs. 8(a) through 8(c) are explanatory views showing examples of arrangement of combination-type stoppers;
Fig. 9 is a cross section showing a first example of a cell observation support instrument that can be used for carrying out the present invention;
Fig. 10 is a cross section showing a second example of a cell observation support instrument that can be used for carrying out the present invention;
Fig. 11 is a cross section showing a third example of a cell observation support instrument that can be used for carrying out the present invention;
Fig. 12 is a cross section showing a fourth example of a cell observation support instrument that can be used for carrying out the present invention;
Fig. 13 is a cross section showing a fifth example of a cell observation support instrument that can be used for carrying out the present invention;
Figs. 14(a) and 14(b) are explanatory views showing observation photographs before and after introduction of cells in an example using the cell observation support instrument of the embodiment;
Figs. 15(a) and 15(b) are explanatory views showing observation photographs before and after introduction of cells in an example using the cell observation support instrument of the embodiment;
Figs. 16(a) and 16(b) are explanatory views showing observation photographs before and after introduction of cells in an example using the cell observation support instrument of the embodiment;
Figs. 17(a) and 17(b) are explanatory views showing observation photographs before and after introduction of cells in an example using the cell observation support instrument of the embodiment;
Figs. 18(a) and 18(b) are explanatory views showing observation photographs before and after introduction of cells in an example using the cell observation support instrument of the embodiment;
Figs. 19(a) and 19(b) are explanatory views showing observation photographs before and after introduction of cells in an example using the cell observation support instrument of the embodiment;
Fig. 20(a) is an enlarged view of the stopper shown in Fig. 4(e), and Fig. 20(b) is an explanatory view showing an arrangement of a plurality thereof; and
Fig. 21 is a block diagram showing an example of a configuration of a cell measurement system that can be used for observation by the cell observation support instrument of the invention.

### Explanation of Symbols

100 ... cell observation support instrument, 110 ... cell-containing space (channel), 111 ... source area, 112 ... drain area, 113, 113a ... first liquid reservoir space, 114, 114a2 ... second liquid reservoir space, 113b ... third liquid reservoir space, 114b ... fourth liquid reservoir space, 115 ... silicon wafer, 117 ... glass base, 120 ... liquid, 130 ... stimulating substance, 150 ... stopper, 151a, 151b ... column structure, 151c ... slit, 200 ... cell, 310 ... microscope, 320 ... digital camera (CCD camera), 350 .... computer

### Best Mode for Carrying Out the Invention

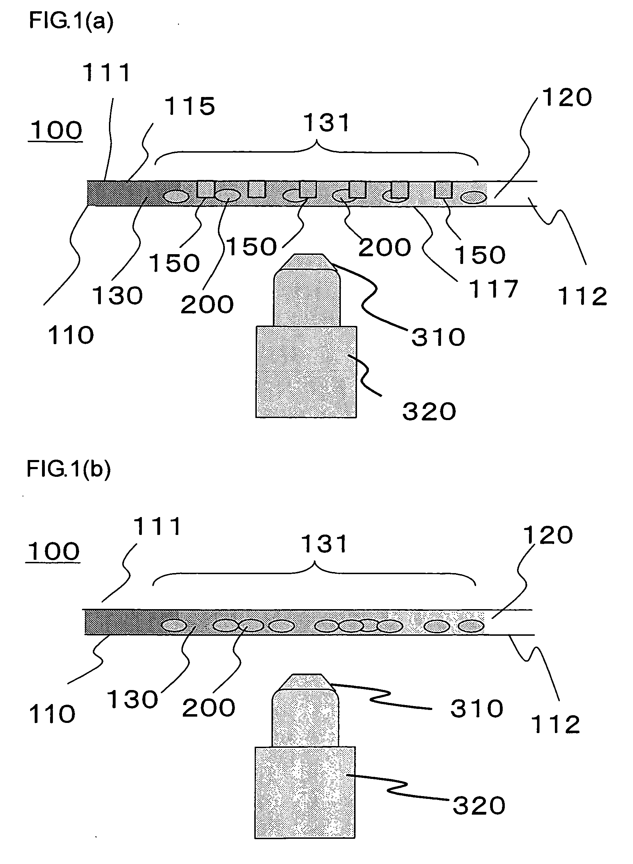

An embodiment of the present invention will be described referring to Figs. 1(a) and 1(b). Fig. 1(a) is a view showing the principle of the present invention. On the other hand, Fig. 1(b) is a view showing a comparative example.

As shown in Fig. 1(a), in the present embodiment, a concentration gradient with respect to a specific substance used for observing reaction of cells is formed in a space where the cells are arranged. To that end, a cell observation support instrument 100 forms a cell-containing space 110 as a space for containing cells. Fig. 1(a) shows the structure of the cell-containing space 110 schematically to explain the principle. Structure details will be described later.

The cell-containing space 110 is filled with liquid 120 harmless to cells. Cells 200 to be observed are arranged and dispersed as a plurality of cells (cell groups) in the liquid 120. In this state, a substance (hereinafter, referred to as stimulating substance) 130 used for observing reaction of the cells is injected from a source area 111 on one side of the cell-containing space 110. In this example, a concentration gradient 131 is formed in the cell-containing space 110 such that the concentration of the stimulating substance decreases from the side of the source area 111 to the side of the drain area 112. For example, a solution, in which the stimulating substance 130, such as a compound, is dissolved, is injected, and an image of a state of each cell 200 is taken by a digital camera (for example, a CCD camera) 320 through a microscope 310 in a state such that a concentration gradient 131 of the specific stimulating substance 130 is formed in the cell-containing space 110. In the present embodiment, a plurality of cell-containing spaces 110 (for example, twelve) are formed on a silicon wafer by using a photolithographic technique. In each cell-containing space 110, a response of a cell in each position of a concentration gradient is measured. By this method, actions of a compound on cells can be compared easily.

Responses of cells to a stimulating substance differ depending on concentrations of the stimulating substance. The responses of the cells may envisage various cell phenomena such as manifestation of a gene, morphological change, release of a physiologically active substance and the like. Sometimes, as such a response, cells present some feature quantity that can be observed from the outside. Using this characteristic, the present invention extracts a feature quantity presented by a cell group by analyzing an image of the cell group, in order to obtain information on responses of cells to a stimulating substance. In the present invention, a concentration gradient is formed in the same image, and thus, it is not necessary to carry out a troublesome trial-and-error task of repeating experiments. Responses at different concentrations can be observed in one experiment.

Alternatively, as another embodiment, the liquid 120 in the cell-containing space 110 can be made to flow continuously, intermittently, or in single bursts, by controlling the liquid, for example, by applying or reducing pressure from the source area 111 or 112. As a result, the liquid around the cells is circulated, and the cells can be observed for a long time. Alternatively, by mixing a suitable substance in the liquid flowing in the cell-containing space, it is possible to provide a stimulus by the substance to the cells at a designated time. According to the present invention, it becomes possible to obtain and analyze images of cell groups also in these cases.

Now, Fig. 1(a) is compared with Fig. 1(b). In the cell observation support instrument 100 shown in Fig. 1(a), plural stoppers 150 are arranged at suitable intervals in the cell-containing space 110. Consequently, some cells 200 are prevented from moving from their positions by stoppers 150. Fig. 1(a) shows a state in which some cell groups abut stoppers 150 in the course of cells' movement from the source area 111 on the left side of the figure toward the drain area 112 so that their movement towards the source area 112 is prevented.

On the other hand, a cell observation support instrument 100 shown in Fig. 1 (b) is not provided with stoppers 150. In other points, the cell observation support instrument 100 shown in Fig. 1(b) is the same as the one shown in Fig. 1(a). By comparing the two instruments, it is found that movement of some cells 200 is prevented by the stoppers 150 in the cell observation support instrument shown in Fig. 1(a), so that cell distributions in the cell-containing spaces 110 are different from each other.

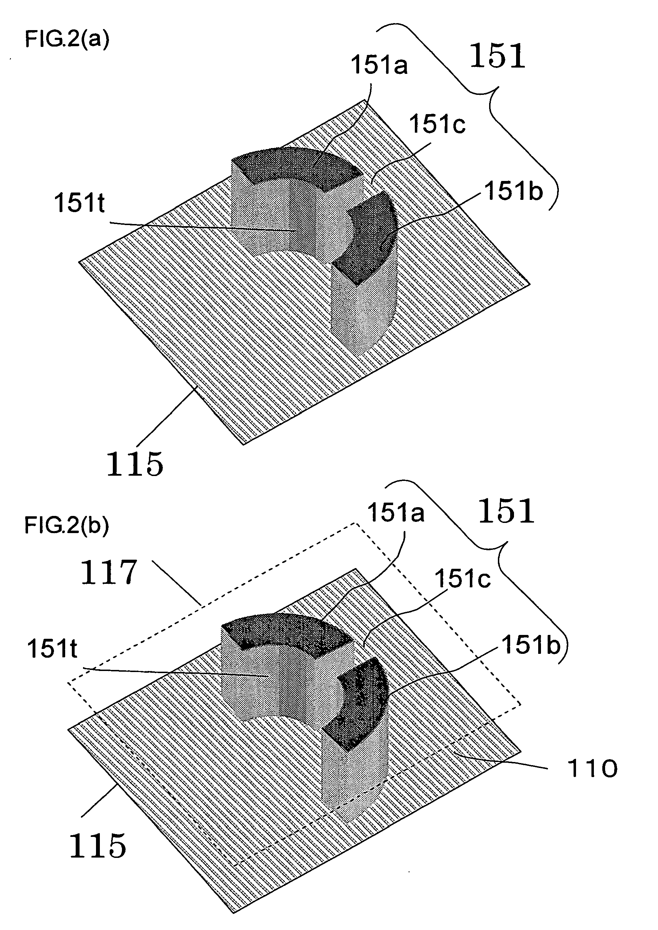

Next, a structure of a stopper will be described referring to Fig. 2(a) through Fig. 3(c). Here, as shown in Fig. 1(a), the present embodiment takes an example where observation is made from the bottom side of the cell observation support instrument 100 by using a microscope. However, to facilitate illustration, a stopper will be described in a state in which its bottom surface is directed upward.

As shown in Figs. 2(a) and 2(b), the stoppers 150 are placed between a first surface member 115 and a second surface member 117 opposed to each other. In the present embodiment, the stoppers 150 are provided on the first surface member 115. The second surface member 117 is positioned above the stoppers 150, keeping a small space from the stoppers 150. The first surface member 115 is made from, for example, a semiconductor wafer, specifically a silicon wafer. A silicon wafer is engraved by using a processing technique such as etching to form an engraved part, leaving the stoppers 150 and the other projections. This engraved part becomes a substantial part of the cell-containing space 110. Thus, in the present embodiment, the stoppers 150 are formed integrally with the first surface member 115. Clearly, the stoppers 150 may be formed separately from both the first and second surface members 115 and 117, and fixed by attachment to one surface member, for example, the first surface member 115.

Any of the first surface member 115, the second surface member 117 and the stoppers may be made of another material. For example, glass or plastic may be cited. It is sufficient that either the first and second surface members 115 and 117 is transparent so as to make optical observation possible.

As shown in Figs. 2(a) and 2(b), each stopper comprises column structures 151a and 151b for holding a cell, and a slit 151c positioned between the column structures 151a and 151b. The slit 151c forms a liquid-passing part having a width such that a cell cannot pass through. The height of the column structures 151a and 151b is equal to or less than the distance between the first and second surface members 115 and 117. When the height of the column structures 151a and 151b is less than the distance between the first and second surface members 115 and 117, it is difficult to prevent liquid from flowing in the cell-containing space 110. Further, the distance between the first and second surface members 115 and 117 is of a similar range as, or is larger than, the diameters of the cells. Further, the column structures 151a and 151b are curved as a whole, as seen in a plane parallel to the upper surface of the first surface member 115. The curve is concave toward the upstream side of the cell flowing direction. It is supposed that the end-to-end dimension of the column structures 151a and 151b, i.e., for example, the length of the curved arc or the chord, is about enough to exceed the supposed diameter of a cell. As a result, a cell holding area 151t, that can trap a cell and does not easily release the trapped cell, is formed. The slit 151c is formed to have a width such that a cell cannot easily pass but liquid can pass. It is not necessary that the cross section of the opening of the slit 151 be rectangular.

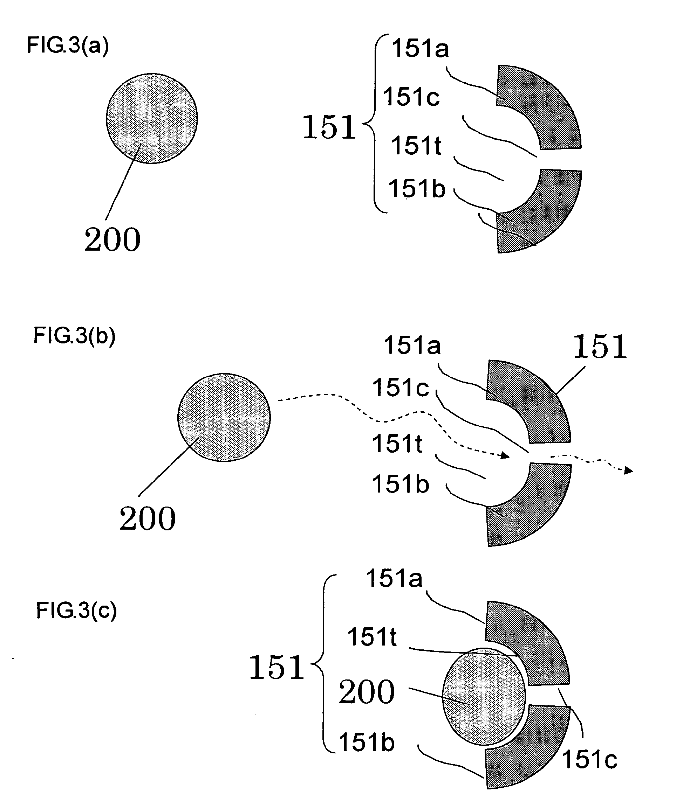

In this way, flow of liquid is ensured, while a cell is easily trapped. In other words, as shown in Figs. 3(a) through 3(c), when a cell suspension is passed through a stopper 151, liquid can pass the slit 151c of the slit 151 but a cell 200 cannot. As a result, the cell 200 is drawn to the slit 151c and held by the stopper 151. In this case, the cell moves with the flow of the liquid, approaches the column structures 151a and 151b, and the liquid between the cell and the column structures 151a and 151b flows outward through the slit 151c even when the cell is at the point of touching the column structures 151a and 151b. Thus, the liquid does not prevent trapping of the cell, and the cell is easily trapped.

On the other hand, when one cell is trapped by a stopper 150, then the cell blocks the slit 151c, and the flow of the liquid to the cell holding area 151t of the stopper 151 is interrupted to some degree. Thus, this has an effect of additionally inhibiting a second cell from being drawn to the stopper 151 in question. As a result, this helps in placing one cell in each stopper. Depending, however, on the shape of a stopper and sizes of cells, it is possible that plural cells are trapped in one stopper.

The liquid passage part is not limited to the above-described slit, and may be formed as a through-hole, a notch, or the like.

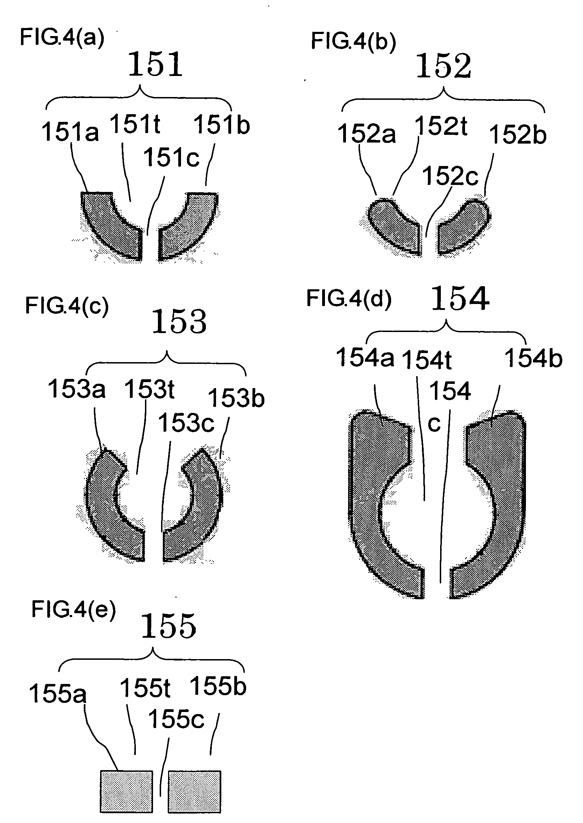

Next, variations of the structure of the stopper will be described referring to Figs. 4(a) through 5(b).

A stopper shown in Fig. 4(a) is the same as those shown in Figs. 2(a) through 3(c), and has a structure like open hands for containing an object. In this case, a standard shape is a semicircle.

A stopper 152 shown in Fig. 4 (c) is characterized in that its cell holding area 152t is shallower than the cell holding area 151t of the stopper 151 shown in Fig. 4(a). As a result, the stopper can have a smaller base area. As regards the structure, the stopper comprises column structures 152a and 152b and a slit 152c.

A stopper 153 shown in Fig. 4(c) comprises column structures 153a and 153b and a slit 153c, and thus the basic structure is the same as the one shown in Fig. 4(a). The column structures 153a and 153b of the stopper 153 of this example, however, have a longer end-to-end dimension, and are more deeply curved. Thus, it is favorable in that a once-trapped cell is not easily released. However, it is possible that plural cells are trapped owing to the larger cell holding area 153t.

A stopper 154 shown in Fig. 4(d) comprises column structures 154a and 154b and a slit 154c, and the basic structure is the same as the one shown in Fig. 4(c). The stopper 154 of this example, however, is longer in its end-to-end dimension, being curved still more deeply. Accordingly, an inlet for a cell is narrow, and favorably, a trapped cell is hardly released.

A stopper 155 shown in Fig. 4(e) comprises column structures 155a and 155b and a slit 155c, and thus the basic structure is the same as the one shown in Fig. 4(a). The stopper 155 of this example, however, is characterized in that the column structures 153a and 153b are not curved. Being non-curved, trapping of a cell does not much depend on the size of the cell. Thus, the stopper 155 is very versatile, being applicable to multiple kinds of cells of different sizes. On the other hand, a trapped cell is easily released. This, however, is advantageous in that cleaning is easy because a cell is easily released at the time of cleaning. Further, since the area in which the column structures 153a and 153b are in contact with a cell is small, there is also an advantage in that the surface of the cell can be observed easily.

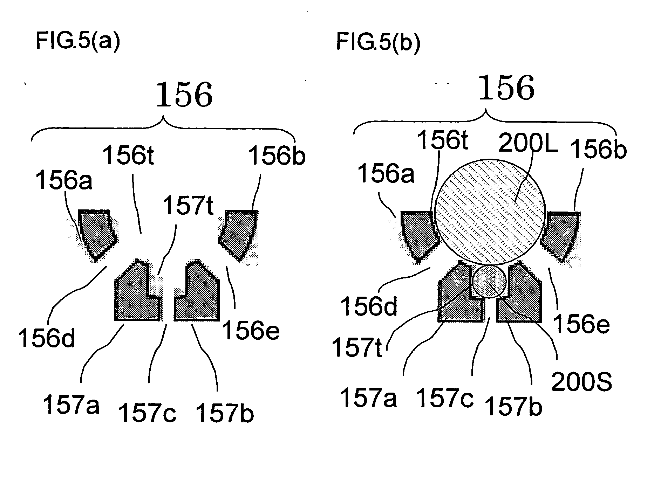

Next, an example of a two-layer stopper shown in Figs. 5(a) and 5(b) will be described. The above-described stoppers are examples for which it is assumed that the cell holding areas 151t through 155t each hold or trap one cell. In some observations, however, cells of different sizes are mixed. For example, some times an experiment is conducted to fuse a cell with another cell. In such a case, a stopper is required to trap a plurality of cells successively, i.e. in a state in which two cells can get touch. The stopper shown in Figs. 5(a) and 5(b) is provided to that end.

This stopper comprises a second layer stopper 157 and a first layer stopper 156 positioned on the inlet side of the second layer stopper 157. The first layer stopper 156 has a larger width on its inlet side, and its outlet is flared. The second layer stopper 157 is placed behind the outlet of the first layer stopper 156. The width of the second layer stopper 157 is smaller than the width of the first layer stopper 156.

The second layer stopper 157 comprises column structures 157a and 157b and a slit 157c, and its basic structure is the same as the one shown in Fig. 4(a). The stopper 157 of this example, however, is characterized in that the column structures 157a and 157b are each bent into an L-shape. This is to facilitate arranging the first layer stopper 156 and forming slits 156d and 156e. The second layer stopper 157 has a structure that can trap a relatively small-sized cell 200S (See Fig. 5(b)).

On the other hand, the first layer stopper 156 comprises column structures 156a and 156b and the slits 156d and 156e, and its basic structure is the same as the one shown in Fig. 4(a). However, the width is very large so that it can allow passage to a small cell 200S and also trap a large cell 200L. The first layer stopper 156 has two slits 156d and 156e. That is, as a result of providing the second layer stopper 157, the slits 156d and 156e are formed between the column structures 156a and 157a and between the column structures 156b and 157b.

As shown in Fig. 5(b), by using this two-layer stopper, the cell holding area 157t can trap a small-sized cell 200S first, and then the cell holding area 156t can trap a large-sized cell 200L. Thus, both cells can touch, to enable observation of an effect such as fusion.

Next, a distribution pattern is described when a plurality of stoppers are arranged on the first surface member 115.

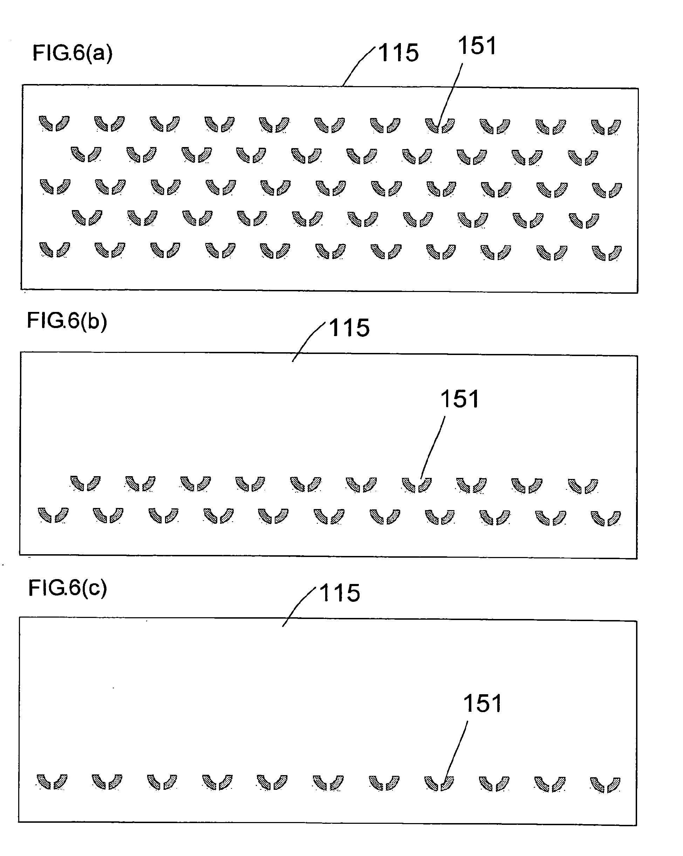

Fig. 6(a) shows a pattern of stoppers 151 of Fig. 4(a) arranged in a staggered array of a plurality of rows and a plurality of columns. All stoppers 151 as elements of the pattern are oriented in the same direction. This is because it is assumed that the liquid is made to flow in one direction. The staggered array arrangement is used for trapping cells efficiently by reducing the number of cells that are not trapped and escape from the stoppers 151.

As shown in Fig. 6(a), by arranging the stoppers 151 on the whole surface of the first surface member 115, the instrument can be used for detecting various cell stimulus responses. For example, in cases where a concentration gradient is provided as shown in Fig. 1(a), a plurality of cells can be fixed at each position in the concentration gradient so that effects of the concentration gradient on cells can be examined at one time. Further, by arranging two-layer stoppers 156 as shown in Fig. 5(a) mentioned above, it is possible to observe effects of concentrations of a stimulating substance on fusion of cells at one time.

Figs. 6(b) and 6(c) show examples where stoppers 151 are reduced in number and arranged concentratedly in a position on the source area side. Such stopper arrangements are suitable when it is desired to observe a state of cells moving in a specific direction after placement of the cells. To take an example, the case of examining effects of a chemotactic factor may be cited.

The distributions of Figs. 6(a) through 6(c) are examples of using the stoppers 151. This is not restrictive. It is possible to arrange stoppers of other structure, for example the stoppers 152 to 156, depending on the cell size or nature. Further, in the case where cells of different sizes are mixed, it is possible to use mixed stoppers suitable for the respective sizes. Similarly, it is also possible to select structures of stoppers depending on ways of cell deformation and to mix the selected-types of stoppers.

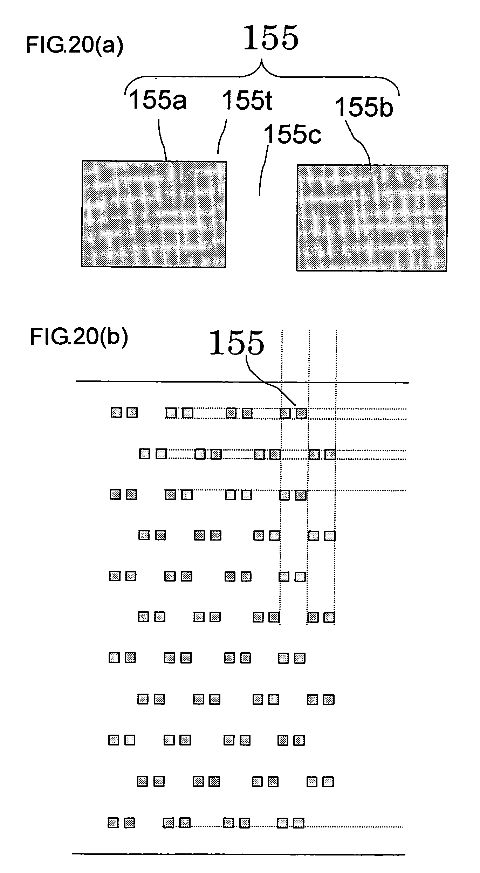

As shown in Fig. 20(b), it is also possible to arrange the stoppers having the shape shown in Fig. 20(a) in a staggered array.

Next, examples of arrangement of stopper combinations each obtained by combining a plurality of stoppers will be described.

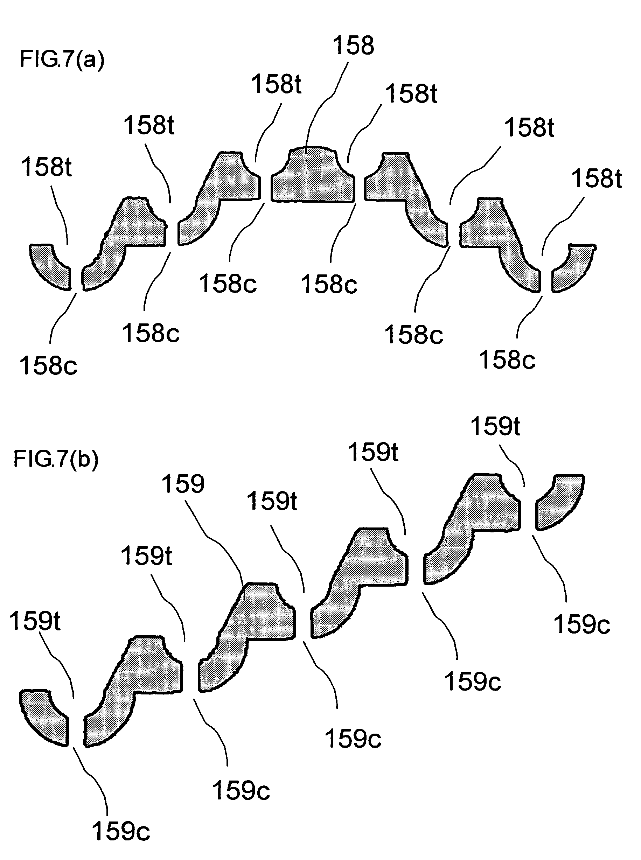

An example shown in Fig. 7(a) uses, as a basic element, a stopper 158 of the same structure as that of the stopper shown in Fig. 4(a), and is formed by combining six of such elements. As a result, six cell holding areas 158t and six slits 158c are arranged in a row forming an arch shape. The stopper combination 158 of this embodiment is not a simple combination of element stoppers but a combination having a curved shape as a whole to form a shape referred to as the arch shape.

An example shown in Fig. 7(b) uses as a basic element a stopper 159 of the same structure as that of the stopper shown in Fig. 4(a), and is formed by combining five of such elements. As a result, five cell holding areas 158t and five slits 158c are arranged in a slanted row. The stopper combination 158 of this embodiment is not a simple combination of element stoppers but is characterized in that element stoppers are combined to be slanted as a whole.

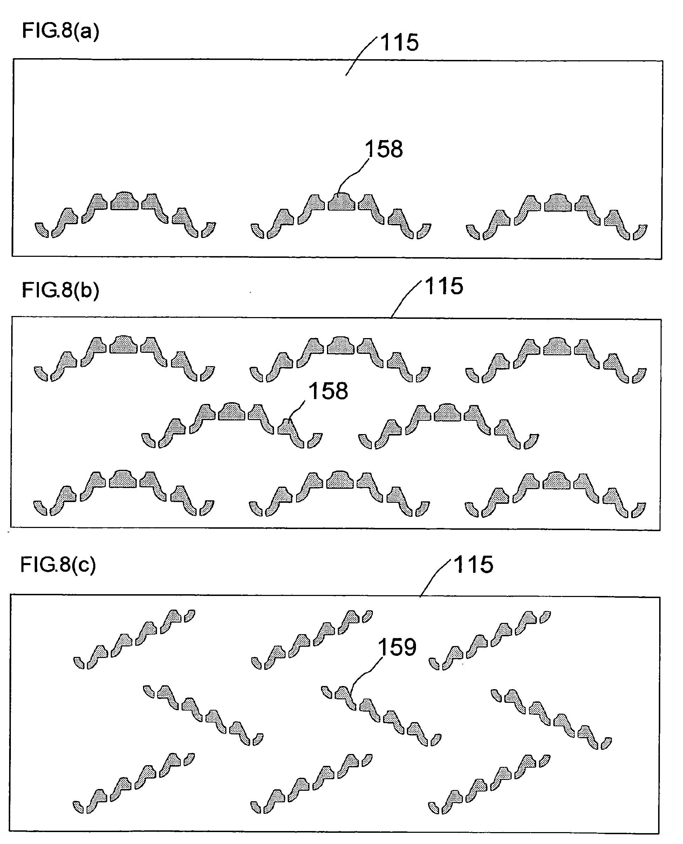

Figs. 8(a) and 8(b) show examples where plural stopper combinations 158 are arranged. Further, Fig. 8(c) shows an example where plural stopper combinations 159 are arranged.

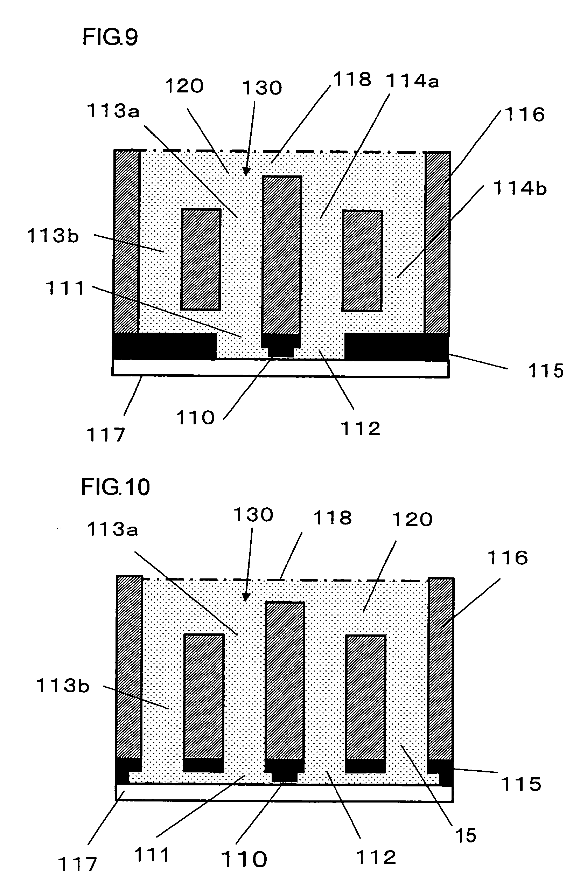

The cell-containing space 110 can be realized specifically in various forms of cell observation support instruments 100 as shown in Figs. 9 to 13. An object of the cell-containing space 110 is to arrange cell groups in a dispersed manner on a flat surface so that the cell groups can be observed through a microscope or the like, from the outside. To that end, the cell-containing space 110 has a flat structure. The thickness of the cell-containing space 110 is approximately the size of a cell, for example. In the examples shown in Figs. 9 to 13, illustration of a stopper is omitted since the cell-containing spaces are minute.

In the example shown in Fig. 9, by using the photolithography technique, a silicon wafer as the first surface member 115 is provided with: a flat engraving that becomes the cell-containing space 110; an engraving that is a source area 111 positioned at one end of the cell-containing space 110 and is used for pouring a stimulating substance into the cell-containing space 110; and an engraving that becomes a drain space 112 positioned at the other end of the cell-containing space 110 and used for discharging the stimulating substance from the cell-containing space 110. The silicon wafer (i.e. the first surface member) 115 is placed on a glass base as the second surface member 117. As a result, the engraved spaces are placed between the silicone wafer (the first surface member) 115 and the glass base (the second surface member) 117 and form the cell-containing space 110, the source area 111 and the drain area 112, respectively. The cell-containing space 110 is formed to provide a flat space (channel) having a depth sufficient to prevent cells from overlapping each other. The source area 111 and the drain area 112 are named, for the sake of convenience, the injection side and the discharge side for a stimulating substance. In the case of a symmetrical cell observation support instrument, it does not matter which side is the source area or the drain area.

On the upper surface side of the silicon wafer (the first surface member) 115, a liquid reservoir formation member 116 is provided. The liquid reservoir formation member 116 is formed of, for example, a metal such as stainless steel. The liquid reservoir formation member 116 forms a first liquid reservoir space 113a, a third liquid reservoir space 113b, a second liquid reservoir space 114a, and a fourth liquid reservoir space 114b. The first and third liquid reservoir spaces 113a and 113b communicate with the source area 111. On the other hand, the second and fourth liquid reservoir spaces 112 and 114 communicate with the drain area 112. Owing to this structure, liquid 120 can freely flow between the first and third liquid reservoir spaces 113a, 113b and the second and fourth liquid reservoir spaces 114a, 114b, through the cell-containing space 110. However, in the present embodiment, it is preferable for observation that the liquid itself is not made to flow. Thus, in the upper portion of the liquid reservoir formation member 116, a communication part 118 is provided for communication between the first and third liquid reservoir spaces 113a, 113b and the second and fourth liquid reservoir spaces 114a, 114b. By feeding the liquid 120 until it reaches the communication part 118, it is possible to suppress occurrence of flow of the liquid owing to pressure difference.

Cells, a stimulating substance, and the like are injected using a syringe or the like. At that time, the injection needle is made to reach the source area 111 to ensure injection into the cell-containing space 110. By sucking the liquid 120 from the source area 112 after the injection of cells, it is possible to introduce the injected cells into the cell-containing space 110. Further, the cells are fixed in the cell-containing space 110 if suitable stoppers exist in the cell-containing space 110.

Fig.10 shows a cell observation support instrument 100 having a structure that is approximately equivalent to the structure shown in Fig. 9. This cell observation support instrument 100 has a structure similar to that shown in Fig. 9 except for an engraving structure formed in the silicon wafer as the first surface member 115, and it functions similarly.

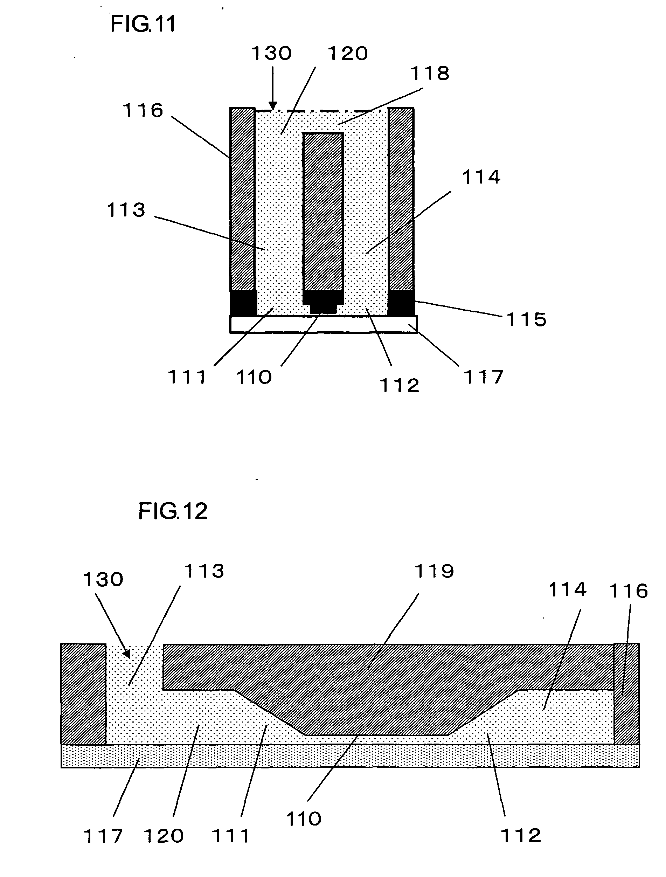

The structure of a cell observation support instrument shown in Fig. 11 does not have the third liquid reservoir space 113b and the fourth liquid reservoir space 114b provided in the cell observation support instruments shown in Figs. 9 and 10. That is, this structure includes the first liquid reservoir space 113, the second liquid reservoir space 114, the source area 111, the cell-containing space 110, and the drain area 112.

In the cell observation support instruments 100 of Figs. 9 and 10, the third liquid reservoir space 113b and the fourth reservoir space 114b are provided in order to absorb liquid fluctuation at injection. In particular, in the case where a stimulating substance is injected at a point distant from the source area 111 using a syringe or the like, the third and fourth liquid reservoir spaces 113b, 114b perform a function of releasing a part of pressure that is generated when the stimulating substance is pushed out.

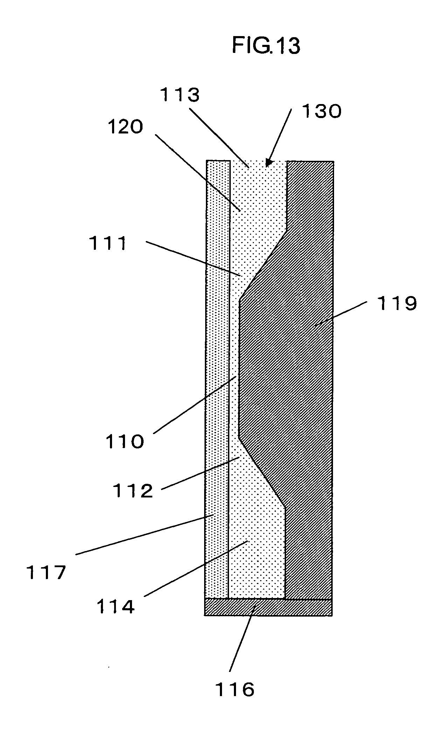

Cell observation support instruments shown in Figs. 12 and 13 have a different type from those shown in Figs. 9 to 11. That is to say, the cell observation support instruments shown in Figs. 12 and 13 are different in the structure of the liquid reservoir spaces and in the way of forming the cell-containing space 110. The first liquid reservoir space 113 is opened and used for injection of liquid, cells, a stimulating substance, and the like. On the other hand, the second liquid reservoir space 114 is not opened. Further, as the material 119 used for forming the cell-containing space 110, stainless steel, not silicon, is used. Owing to this construction, production of the cell observation support instrument is simplified and production cost is reduced.

Fig. 12 shows an example of a horizontal type, i.e. a type to be positioned horizontally. On the other hand, an example shown in Fig. 13 is a vertical type. When the example shown in Fig. 13 is used for sticky cells, the cells adhere to the glass base 117 and do not drop.

In cases of the types shown in Figs. 12 and 13, cells are injected into the source area 111, and then the cells can be introduced into the cell-containing space 110 by using gravity or a centrifugal force.

Here, a concentration gradient will be described.

In cases of the above-described cell observation support instrument shown in Fig. 11, the space comprising the cell-containing space (channel) 110, the first liquid reservoir space 113, and the second liquid reservoir space 114, is filled with a suitable liquid. Further, a solution including a substance for forming a concentration gradient is injected into one of the ducts of the first and second liquid reservoir spaces 113, 114. As a result, the substance diffuses in the cell-containing space 110 from the source area 111 toward the drain area 112. When the liquid 120 is filled also in the communication part 118 in the upper portion, it is possible to significantly reduce turbulence of the concentration gradient owing to violent movement of the liquid in the cell-containing space 110 due to the effects of vibration or tilting. In this way, the concentration gradient can be stably maintained.

As shown in Figs. 9 and 10, it is possible to use a cell observation support instrument in which the first liquid reservoir space 113a is provided with the third liquid reservoir space 113b as a branch, and the second liquid reservoir space 114a is provided with the fourth liquid reservoir space 114b as a branch. In such cases, a liquid including the desired stimulating substance 130 is introduced from the first liquid reservoir space 113a or the second liquid reservoir space 114a. In cases where the liquid of the stimulating substance is injected from the side of the first liquid reservoir space 113a, the stimulating substance diffuses from the source area 111 through the cell-containing area 110 toward the drain area. On the other hand, in cases where the liquid of the stimulating substance is introduced from the side of the second liquid reservoir space 114a, the drain area 112 shown in the figure is substantially a source area, and the source area 111 on the opposite side is a drain area. Thus, in the cases of the cell observation support instruments shown in Figs. 9, 10 and 11, the source area and the drain area are expediential names.

A concentration gradient can be formed also using the cell observation support instrument shown in Fig. 12 or 13. In that case, a liquid including a stimulating substance 130 is introduced from the opened first liquid reservoir space 113, so that a concentration gradient of the stimulating substance 130 is formed from the source area 111 through the cell-containing space 110 toward the drain.

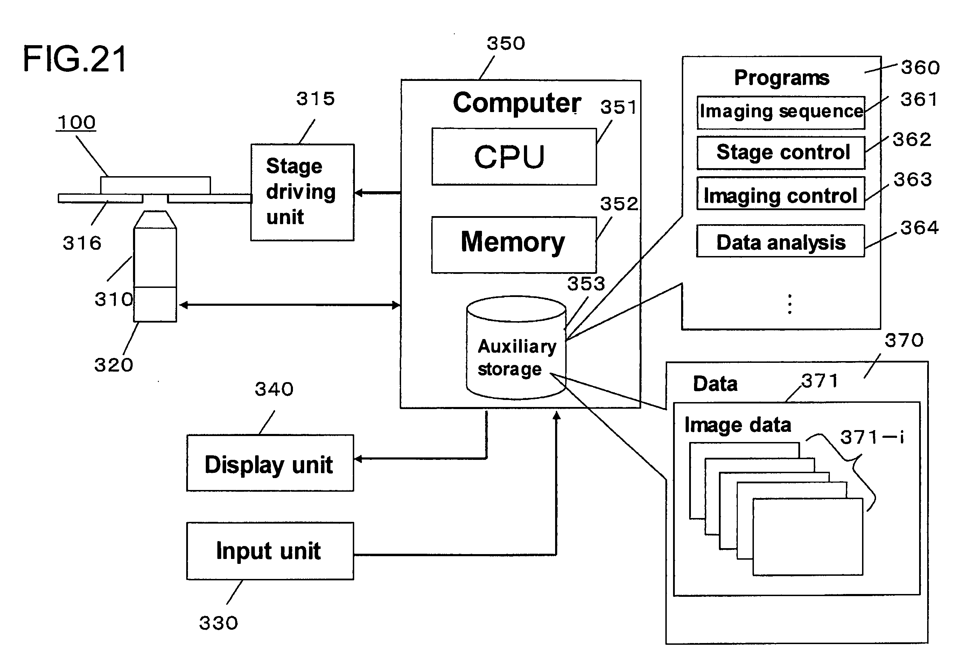

Fig. 21 shows an example of a configuration of a cell measurement system that is used for observation by the cell observation support instrument of the present invention. The cell measurement system shown in Fig. 2 comprises an image pickup apparatus for taking an image of cells received in the cell-containing space 110 and an information processing apparatus for performing image processing for processing the image taken to obtain desired information.

In the present embodiment, the image pickup apparatus comprises a microscope 310, a CCD camera 320, a stage 316, a stage driving unit 315, and an information processing unit 350. On the other hand, the information processing apparatus comprises an input unit 330, a display unit 340, and a computer 350.

The computer 350 comprises a central processing unit (CPU) 351, a memory 352, and an auxiliary storage device 353. The auxiliary storage device 353 stores operation programs 360 for the CPU 351 and data 370. The programs 360 include an OS (not shown) as well as an imaging sequence 361 for controlling measurement operations in the cell measurement system, a stage control 352 for controlling the stage, imaging control 363 for controlling imaging, and data analysis 364 for analyzing obtained image data. These programs have been installed onto the auxiliary storage device 353 from a storage medium, a network, or the like. Image data 371 are representative of the data.

Image pickup of cells is performed by preparing cell groups to be observed by the cell observation support instrument and performing measurement under control of the computer 350. Measurement is performed according to the previously determined imaging sequence 361. That is, according to the stage control program 362, the computer 350 controls the stage driving unit 315 for positioning to take images at a plurality of positions (for example, twelve) of the cell observation support instrument at a given timing, for example, at one-minute intervals. The imaging sequence 361 not only performs positioning by the XY stage 316 but also instructs the CCD camera 320 to take images of the cell groups in the cell-containing space 110 at the given timing according to the imaging control program 363. Then, images taken by the CCD camera 320 are inputted into the memory 352 at predetermined timing.

The inputted image data are subjected to analysis processing by the data analysis program 64. First, the data of the images taken are stored together with image taking conditions in the auxiliary storage device 353.

After that, obtained images are subjected to designated processing. At that time, it is possible to perform processing to remove images of the stoppers.

Now, examples will be used for further description.

### Example 1

The cell observation support instrument shown in Fig. 9 having the stopper arrangement pattern shown in Fig. 6 was used for trapping cells. The cell observation support instrument 100 is constructed by using a glass base as the second surface member 117 and a silicon wafer as the first surface member 115 and the stoppers.

A liquid that does not have an influence on the cells is injected in advance into the space of the cell observation support instrument. Next, cells are introduced into the source area 111, and sucked by a syringe from the side of the drain area 112. The cells used are mast cells collected from the abdominal cavity of a rat. Under this condition, the cells are observed by the microscope through the second surface member, and images of the cells are taken.

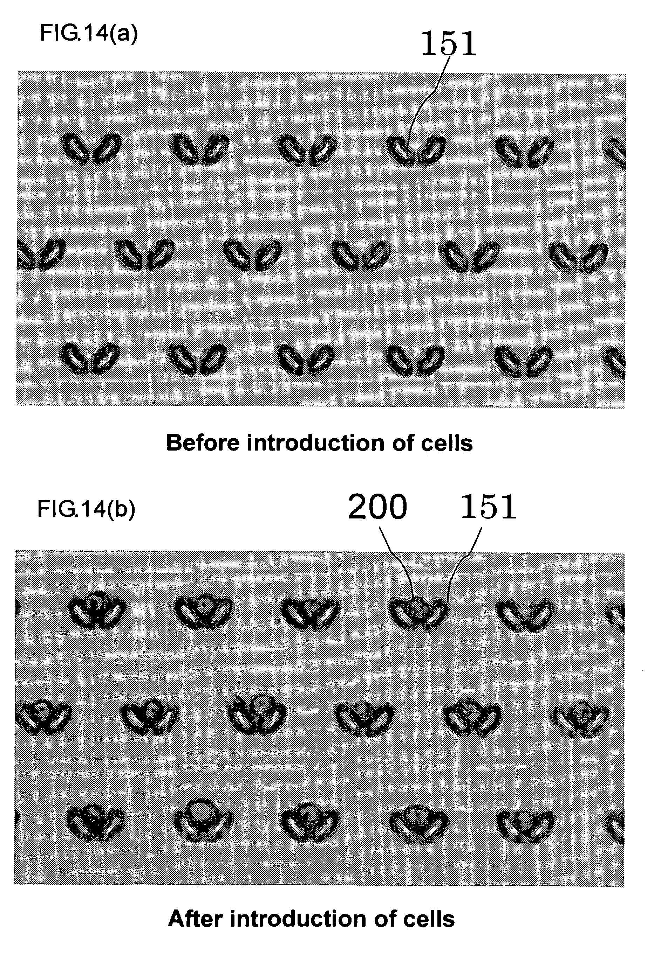

A photograph in Fig. 14(a) shows a state before introducing the cells. Fig. 14(b) shows a state after introducing the cells. It is confirmed that cells are assuredly held by stoppers.

### Examples 2 - 5

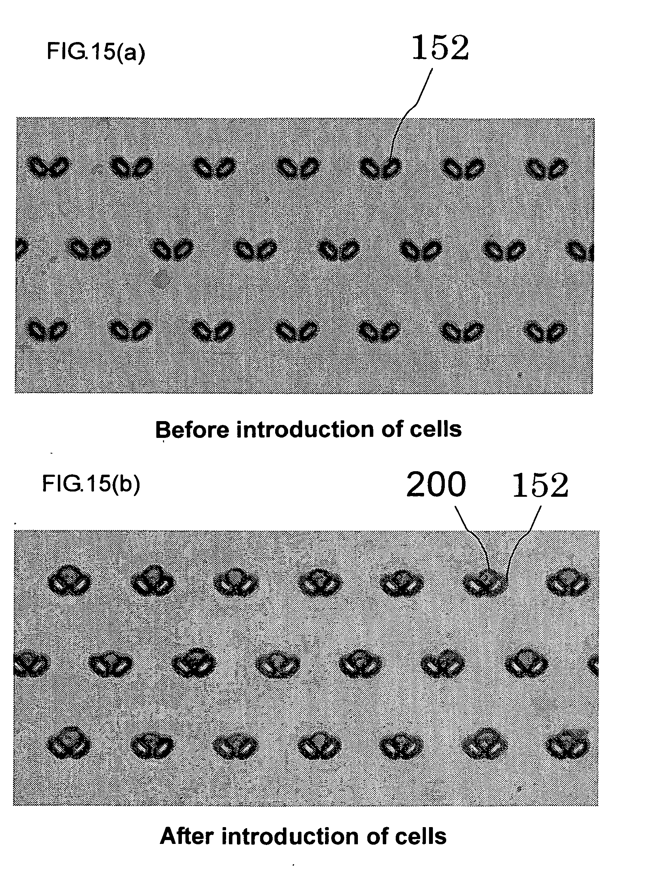

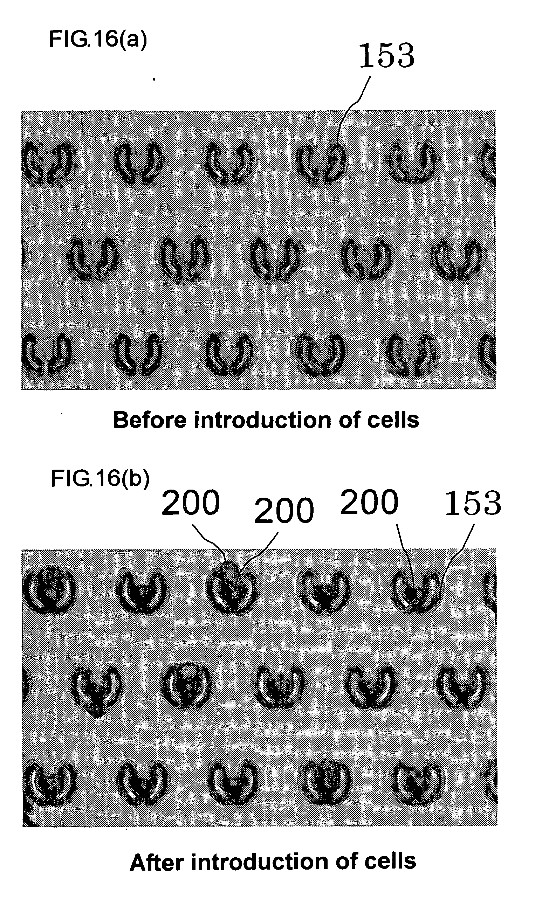

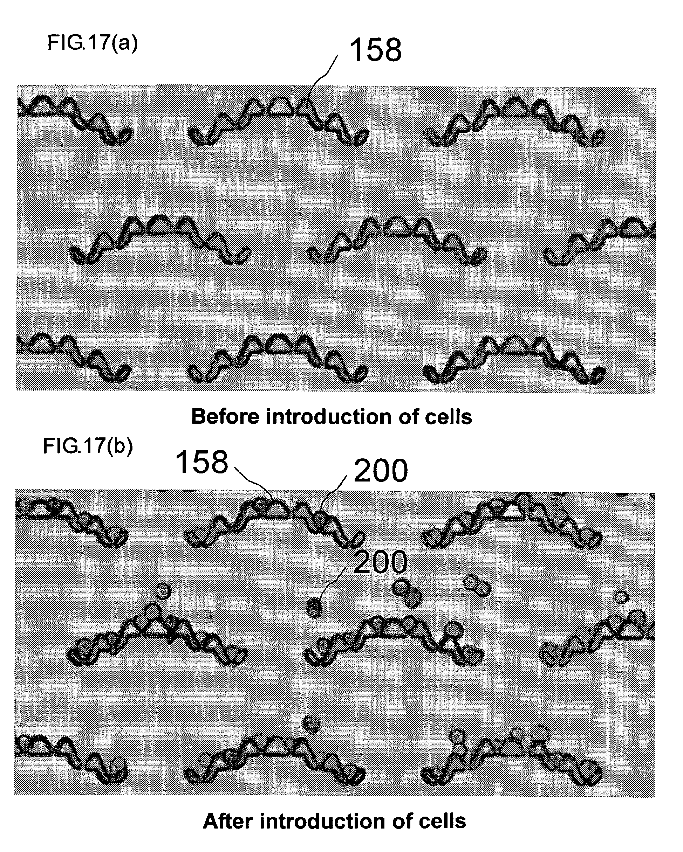

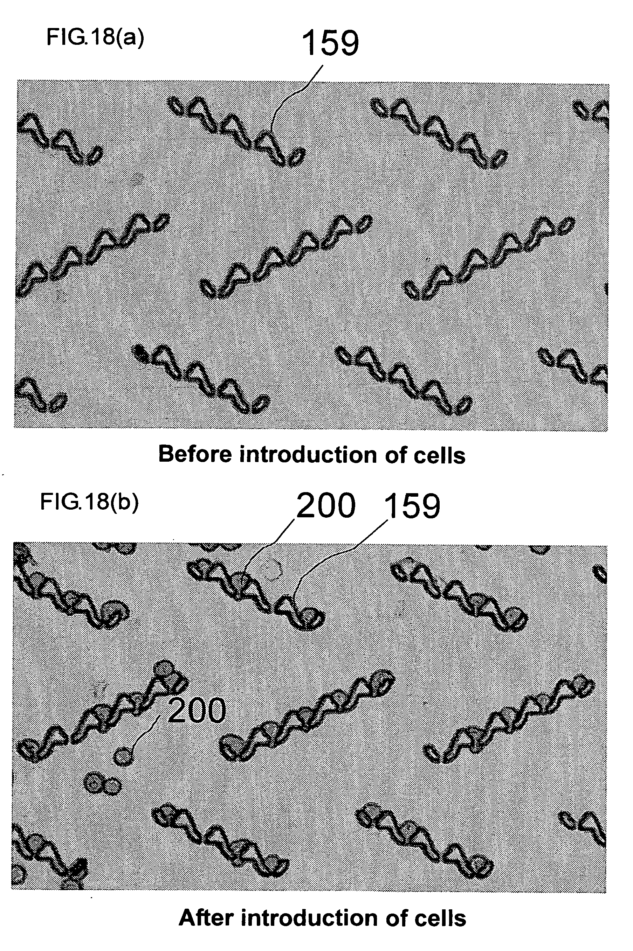

Trapping of cells was examined similarly to Example 1 except for different structures of stoppers. As a result, as shown in Figs. 15(a), 16(a), 17(a) and 18(a) showing states before introducing cells, and Figs. 15(b), 16(b), 17(b) and 18(b) showing stage after introducing cells, it can be seen that each stopper traps a cell or cells.

### Example 3

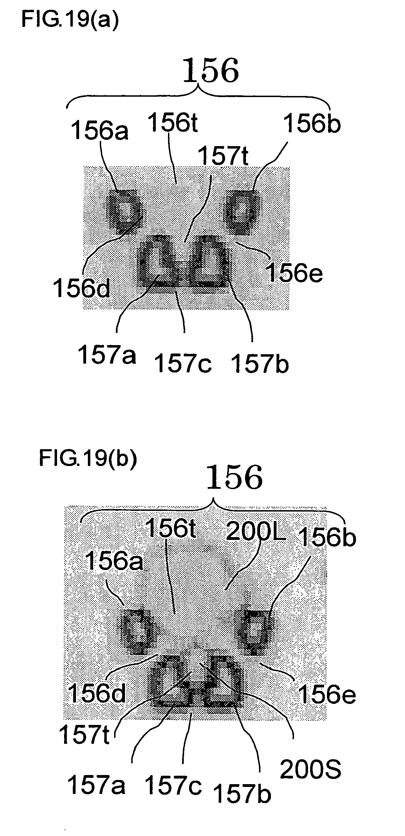

Trapping of cells was examined in cases where the stoppers shown in Fig. 5 (a) were used, and large-sized cells were introduced after introducing small-sized cells. Figs. 19(a) and 19(b) show the result. As shown in Fig. 19(a), no cell was trapped before introduction of cells. On the other hand, as shown in Fig. 19(b), it was confirmed that two-kinds of cells, large-sized and small-sized, were trapped.

As described above, according to the present invention, it is possible to arrange cells easily at positions of stoppers provided on a flat surface. As a result, the following can be expected.

It is possible to directly observe a state of the arranged cells.

It is possible to introduce a compound into an environment where the arranged cells exist.

It is possible to form a concentration gradient of the compound in the environment where the arrange cells exist.

It is possible to easily observe interaction of cells owing to contact among a plurality of cells.

## Claims

1. A cell observation support instrument comprising:
a first surface member and a second surface member, both members being opposed to each other; and
stoppers that are positioned between the first surface member and the second surface member, and prevent movement of cells; wherein
the first surface member and the second surface member form a cell-containing space between opposed surfaces of the first and second surface members, to contain a plurality of cells while allowing movement of the cells;
at least one of the first and second surface members is a member through which an inner side of the cell-containing space can be optically observed from outside; and
one or more of the stoppers are arranged in the cell-containing space.

2. A cell observation support instrument of Claim 1, wherein:
the stoppers each have a liquid passing part having a size that can prevent passing of at least one cell; and
the column structure has a liquid passing part that allows passing of liquid without allowing passing of a cell.

3. A cell observation support instrument of Claim 2, wherein:
the column structure has a curved portion that is concave toward an upstream side of flow of the cells.

4. A cell observation support instrument of one of Claims 2 and 3, wherein:
height of the stoppers equals to a distance between the first surface member and the second surface member.

5. A cell observation support instrument of Claim 4, wherein:
the height of the stoppers is equivalent to cell diameter of the cells to be observed.

6. A cell observation support instrument of any one of Claims 1 - 5, wherein:
the stoppers are arranged in at least one row.

7. A cell observation support instrument of Claim 6, wherein:
the stoppers are arranged in a plurality of rows to form a staggered array.

8. A cell observation method for optically observing cells by filling a cell-containing space formed by opposed first and second surface members, introducing the cells into the liquid, and optically observing the cells through a transparent member from among the first surface member and the second surface member, wherein:
at least one stopper is arranged between the first and second surface members, and prevents movement of the cells; and
a plurality of cells are introduced into the cell-containing space to observe cells whose movement is prevented by any of the stoppers.
